# EUROPÄISCHE PATENTANMELDUNG

(11) **EP 2 546 230 A1**
(43) Veröffentlichungstag der Anmeldung: **16.01.2013**
(21) Anmeldenummer: 11174275.5
(22) Anmeldetag: 15.07.2011
(51) Int. Cl.: C07C 211/27, C08G 59/50, C08G 59/56, C09D 163/00

(54) **Emissionsarmer Härter für Epoxidharze**

(71) Anmelder: Sika Technology AG, 6340 Baar (CH)
(72) Erfinder: Kasemi, Edis, 8046 Zürich (CH); Kramer, Andreas, 8006 Zürich (CH); Stadelmann, Ursula, 8046 Zürich (CH); Burckhardt, Urs, 8049 Zürich (CH)
(74) Vertreter: Sika Patent Attorneys

(57) **Zusammenfassung**

Die vorliegende Erfindung betrifft geruchsarme, niedrigviskose Härter für Epoxidharze umfassend das Amin der Formel (I), 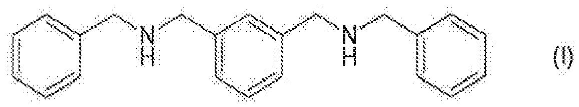 sowie eine Methode zum Verdünnen von Härtern für Epoxidharze durch Zugabe des Amins der Formel (I). Diese Härter härten mit Epoxidharzen schnell und ohne Blushing zu Filmen von hoher Härte bei geringer Sprödigkeit, auch ohne nicht einbaubare Verdünner wie Benzylalkohol. Sie sind insbesondere geeignet für emissionsarme Beschichtungen.

## Beschreibung

### Technisches Gebiet

Die Erfindung betrifft das Gebiet der Amine und ihre Verwendung als Härter für Epoxidharze, sowie Amine enthaltende Epoxidharz-Zusammensetzungen und ihre Verwendung, insbesondere als Beschichtung.

### Stand der Technik

Epoxidharz-Zusammensetzungen sollen eine Reihe von Eigenschaften aufweisen, um als Beschichtung von hoher Qualität verwendbar zu sein. Einerseits sollen sie eine niedrige Viskosität aufweisen, damit sie bei Umgebungstemperatur gut verarbeitbar und selbstveriaufend sind, und sie sollen ohne sogenannte Blushing-Effekte schnell aushärten, auch bei feucht-kalten Bedingungen. Als "Blushing" werden Mängel bei der Aushärtung wie Trübungen, Flecken und raue oder klebrige Oberfläche bezeichnet, typischerweise verursacht durch Salzbildung von Aminen mit Kohlendioxid (CO₂) aus der Luft, wobei eine hohe Luftfeuchtigkeit und tiefe Temperaturen Blushing-Effekte begünstigen. Im ausgehärteten Zustand soll die Epoxidharz-Beschichtung eine ebenmässige Oberfläche ohne Trübungen, Flecken oder Krater aufweisen, und sie soll eine hohe Härte bei möglichst geringer Sprödigkeit besitzen, um mechanischer Beanspruchung gut zu widerstehen, was beispielsweise in der Anwendung als Schutzbeschichtung oder als Bodenbelag besonders wichtig ist. Um diese Eigenschaften zu erreichen, werden in Epoxidharz-Beschichtungen nach dem Stand der Technik üblicherweise Verdünner eingesetzt. Solche Verdünner, wie Benzylalkohol oder Phenole, verbessern die Verarbeitbarkeit und reduzieren die Sprödigkeit stark, werden bei der Aushärtung aber nicht in die Harzmatrix eingebaut. Immer wichtiger wird heutzutage aber die Forderung nach emissionsarmen Systemen, die nach der Aushärtung einen geringen Gehalt von durch Verdampfungs- oder Diffusionsprozesse freisetzbaren Substanzen aufweisen. Für emissionsarme Systeme können nicht einbaubare Verdünner deshalb nur in sehr geringer Menge oder gar nicht verwendet werden. Eine weitere Möglichkeit, Epoxidharz-Zusammensetzungen zu verdünnen, ist die Zugabe von niedrigmolekularen Aminen wie beispielsweise Isophorondiamin, Xyiylendiamin oder Dimethylaminopropylamin. Solche niedrigmolekulare Amine sind aber meist geruchsintensiv und stark hautreizend und führen bei feucht-kalten Bedingungen zu Blushing-Effekten.

US 2009/0163676 beschreibt Härter für Epoxidharze enthaltend mindestens ein benzyliertes Polyalkylenpolyamin und mindestens ein weiteres Amin.

US 6'562'934 beschreibt Härter für Epoxidharze enthaltend Umsetzungsprodukte von Diaminen mit Alkenylverbindungen. Die Härter dieser beiden Schriften weisen den Nachteil auf, dass sie mit Epoxidharzen insbesondere bei tiefen Temperaturen langsam aushärten und ohne den Einsatz von nicht einbaubaren Verdünnern zu eher spröden Beschichtungen führen.

### Darstellung der Erfindung

Aufgabe der vorliegenden Erfindung ist es daher, Härter für Epoxidharze zur Verfügung zu stellen, die geruchsarm, niedrigviskos und mit Epoxidharzen gut verarbeitbar sind und auch bei feucht-kalten Bedingungen schnell und ohne Blushing aushärten, auch ohne Anwesenheit von nicht einbaubaren Verdünnern.

Überraschenderweise wurde gefunden, dass Härter nach Anspruch 1 umfassend das Amin der Formel (I) diese Aufgabe lösen. Der Härter ist geruchsarm, niedrigviskos und verträgt sich ausgezeichnet mit Epoxidharzen. Mit diesen härtet er dabei auch bei feucht-kalten Bedingungen schnell aus und bildet Filme, die nicht klebrig sind, einen hohen Glanz aufweisen, frei sind von Trübungen und Oberflächenstörungen und eine überraschend hohe Härte besitzen. Da das Amin der Formel (I) keine primären, sondern lediglich zwei sekundäre Aminogruppen und ein vergleichsweise hohes NH-Equivalentgewicht aufweist, würde der Fachmann langsam aushärtende, klebrige und/oder wenig harte Filme erwarten.

Mit dem Härter nach Anspruch 1 sind emissionsarme Epoxidharz-Systeme zugänglich, welche die Bedingungen für Öko-Gütesiegel, beispielsweise nach Emicode (EC1 Plus), AgBB, DIBt, Der Blaue Engel, AFSSET, RTS (M1) und US Green Building Council (LEED), erfüllen und gleichzeitig hohen Ansprüchen bezüglich Verarbeitungs- und Gebrauchseigenschaften genügen, was mit Härtern aus dem Stand der Technik nicht gelingt.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Härter, geeignet zum Aushärten von Epoxidharzen, umfassend das Amin der Formel (I).

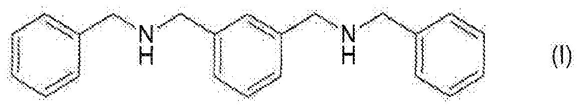

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten. Als "aliphatisch" wird ein Amin bezeichnet, dessen Aminogruppe an einen aliphatischen, cycloaliphatischen oder arylaliphatischen Rest gebunden ist; entsprechend wird diese Gruppe als aliphatische Aminogruppe bezeichnet. Als "aromatisch" wird ein Amin bezeichnet, dessen Aminogruppe an einen aromatischen Rest gebunden ist; entsprechend wird diese Gruppe als aromatische Aminogruppe bezeichnet.

Als "Aminwasserstoff" werden die Wasserstoffatome von primären und sekundären Aminogruppen bezeichnet.

Als "NH-Equivalentgewicht" wird der Gewichtsanteil eines Härters oder eines Amins pro im Härter oder im Amin vorhandenen Aminwasserstoff bezeichnet. Als "nicht einbaubarer Verdünner" wird eine in einem Epoxidharz lösliche und dessen Viskosität sendende Substanz bezeichnet, welche bei der Aushärtung des Epoxidharzes nicht kovalent in die Harzmatrix eingebaut wird.

Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in DIN EN ISO 3219 beschrieben bestimmt wird.

Das Amin der Formel (I) kann besonders vorteilhaft durch die reduktive Alkylierung von 1 ,3-Bis-(aminomethyl)benzol (= meta-Xylylendiamin oder MXDA) mit Benzaldehyd erhalten werden. Dabei wird Benzaldehyd bevorzugt stöchiometrisch in Bezug auf die primären Aminogruppen von 1,3-Bis-(aminomethyl)benzol eingesetzt. Die reduktive Alkylierung wird geeigneterweise in Anwesenheit von Wasserstoff und unter erhöhtem Druck durchgeführt. Sie kann direkt mit molekularem Wasserstoff oder indirekt durch Wasserstoff-Transfer von anderen Reagentien erfolgen. Bevorzugt wird molekularer Wasserstoff verwendet. Dabei werden die Bedingungen vorteilhaft so gewählt, dass einerseits die primären Aminogruppen möglichst vollständig reduktiv alkyliert werden und andererseits die Benzolringe nicht hydriert werden. Bevorzugt wird bei einem Wasserstoff-Druck von 5 bis 100 bar, einer Temperatur von 40 bis 120 °C und in Anwesenheit eines geeigneten Katalysators gearbeitet. Als Katalysator bevorzugt sind Palladium auf Kohle (Pd/C), Platin auf Kohle (Pt/C), Adams-Katalysator und Raney-Nickel, insbesondere Palladium auf Kohle und Platin auf Kohle.

Die Herstellung des Amins der Formel (I) durch reduktive Alkylierung auf die beschriebene Weise ist für die Verwendung als Bestandteil von Härtern für Epoxidharze besonders vorteilhaft, da sehr selektiv primäre Aminogruppen alkyliert werden, während sekundäre Aminogruppen kaum weiter alkyliert werden. Das Produkt aus der beschriebenen Herstellung kann deshalb nach der reduktiven Alkylierung ohne weitere Aufbereitung zur Aushärtung von Epoxidharzen in der beschriebenen Weise verwendet werden.

Das Amin der Formel (I) lässt sich auch auf andere Weise als durch reduktive Alkylierung erhalten, insbesondere durch Umsetzung von 1,3-Bis-(aminomethyl)benzol mit Benzylchlorid oder Benzylbromid in einem geeigneten Verhältnis. Dabei entstehen Reaktionsgemische, welche typischerweise einen erheblichen Anteil an doppelt alkylierten Aminogruppen aufweisen.

Das Amin der Formel (I) ist eine wenig flüchtige, geruchsarme Substanz von niedriger Viskosität. Es weist eine so geringe Reaktivität gegenüber CO₂ auf, dass es - im Gegensatz zu vielen aus dem Stand der Technik bekannten Aminen - an der Luft weder zur Bildung von Krusten noch zu Ausfällungen oder Viskositätserhöhungen neigt. Es zeigt eine ausgezeichnete Verträglichkeit mit anderen Aminen und mit Epoxidharzen.

Härter umfassend das Amin der Formel (I) weisen eine niedrige Viskosität bei einem relativ hohen NH-Equivalentgewicht auf und verdünnen damit Epoxidharze gut. Mit Epoxidharzen härten sie überraschend schnell und ohne Biushhg-Effekte aus, insbesondere auch bei tiefen Temperaturen. Bei flächiger Anwendung entstehen ebenmässige, nichtklebrige Filme von überraschend hoher Härte.

Der erfindungsgemässe Härter, geeignet zum Aushärten von Epoxidharzen, umfasst neben dem Amin der Formel (I) insbesondere zusätzlich mindestens ein Polyamin **A,** welches mindestens drei gegenüber Epoxidgruppen reaktive Aminwasserstoffe aufweist.

Härter umfassend das Amin der Formel (I) in Kombination mit einem Polyamin **A** führen zusätzlich zu einer Beschleunigung der Aushärtung von Epoxidharzen, wobei ausgehärtete Harze erhalten werden, welche eine höhere Härte bei geringer Sprödigkeit aufweisen. Dies ist insbesondere für Anwendungen als Beschichtung vorteilhaft. Härter umfassend das Amin der Formel (I) in Kombination mit einem Polyamin **A** weisen ebenfalls eine niedrige Viskosität auf. Insbesondere ist die Viskosität des Härters auch dann niedrig, wenn das Polyamin **A** eine deutlich höhere Viskosität als das Amin der Formel (I) aufweist. Das Amin der Formel (I) wirkt sich also deutlich verdünnend auf das Polyamin **A** aus, besonders wenn dieses höherviskos ist. Das Amin der Formel (I) erlaubt es somit, ein Polyamin **A** wirkungsvoll zu verdünnen, ohne einen nicht einbaubaren Verdünner oder ein geruchsintensives, zu Blushing-Effekten neigendes Amin zu verwenden. Besonders vorteilhaft ist die Verdünnung für Polyamine **A** mit einer Viskosität oberhalb von 700 mPa·s, insbesondere oberhalb von 1 '500 mPa·s. Durch die Anwesenheit des Amins der Formel (I) werden die Aushärtungsgeschwindigkeit des Härters mit Epoxidharzen und die Härte und Oberflächenqualität solcher Filme überraschenderweise nicht negativ beeinflusst und zusätzlich die Sprödigkeit reduziert.

Als Polyamin **A** geeignet sind insbesondere die folgenden Polyamine:
- aliphatische, cycloaliphatische oder arylaliphatische primäre Diamine, wie insbesondere Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis-(4-aminocyclohexyl)methan (H₁₂-MDA), Bis-(4-amino-3-methylcyclohexyl)methan, Bis-(4-amino-3-ethylcyclohexyl)methan, Bis-(4-amino-3,5-dimethylcyclohexyl)methan, Bis-(4-amino-3-ethyl-5-methylcyclohexyl)methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)bicyclo[2.2.1]heptan (NBDA), 3(4), 8(9)-Bis-(aminomethyl)tricyclo[5.2.1.0^{2,6}]decan, 1,4-Diamino-2,2,6-trimethylcyclohexan (TMCDA), 1,8-Menthandiamin, 3,9-Bis-(3-aminopropyl)-2,4,8,10-tetraoxaspiro[5,5]undecan sowie 1,3- und 1,4-Bis-(aminomethyl)-benzol;
- aliphatische, cycloaliphatische oder arylaliphatische primäre Triamine wie 4-Aminomethyl-1,8-octandiamin, 1,3,5-Tris-(aminomethyl)benzol, 1,3,5-Tris-(aminomethyl)cyclohexan, Tris-(2-aminoethyl)amin, Tris-(2-aminopropyl)-amin und Tris-(3-aminopropyl)amin;
- Ethergruppen-haltige aliphatische primäre Diamine, wie insbesondere Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin und höhere Oligomere dieser Diamine, Bis-(3-aminopropyl)polytetrahydrofurane und andere Polytetrahydrofuran-diamine, sowie Polyoxyalkylen-Diamine. Letztere stellen typischerweise Produkte aus der Aminierung von Polyoxyalkylen-Diolen dar und sind beispielsweise erhältlich unter dem Namen Jeffamine^{®} (von Huntsman), unter dem Namen Polyetheramine (von BASF) oder unter dem Namen PC Amine^{®} (von Nitroil). Insbesondere geeignete Polyoxyalkylen-Diamine sind Jeffamine^{®} D-230, Jeffamine^{®} D-400, Jeffamine^{®} D-2000, Jeffamine^{®} D-4000, Jeffamine^{®} XTJ-511, Jeffamine^{®} ED-600, Jeffamine^{®} ED-900, Jeffamine^{®} ED-2003, Jeffamine^{®} XTJ-568, Jeffamine^{®} XTJ-569, Jeffamine^{®} XTJ-523, Jeffamine^{®} XTJ-536, Jeffamine^{®} XTJ-542, Jeffamine^{®} XTJ-559, Jeffamine^{®} EDR-104, Jeffamine^{®} EDR-148, Jeffamine^{®} EDR-176; Polyetheramine D 230, Polyetheramine D 400 und Polyetheramine D 2000, PC Amine^{®} DA 250, PC Amine^{®} DA 400, PC Amine^{®} DA 650 und PC Amine^{®} DA 2000;
- primäre Polyoxyalkylen-Triamine, welche typischerweise Produkte aus der Aminierung von Polyoxyalkylen-Triolen darstellen und beispielsweise erhältlich sind unter dem Namen Jeffamine^{®} (von Huntsman), unter dem Namen Polyetheramine (von BASF) oder unter dem Namen PC Amine^{®} (von Nitroil), wie insbesondere Jeffamine^{®} T-403, Jeffamine^{®} T-3000, Jeffamine^{®} T-5000, Polyetheramine T 403, Polyetheramine T 5000 und PC Amine^{®} TA 403;
- tertiäre Aminogruppen aufweisende Polyamine mit zwei primären aliphatischen Aminogruppen, wie insbesondere N,N'-Bis-(aminopropyl)-piperazin, N,N-Bis-(3-aminopropyl)methylamin, N,N-Bis-(3-aminopropyl)ethylamin, N,N-Bis-(3-aminopropyl)propylamin, N,N-Bis-(3-aminopropyl)cyclohexylamin, N,N-Bis-(3-aminopropyl)-2-ethyl-hexylamin, sowie die Produkte aus der doppelten Cyanoethylierung und nachfolgender Reduktion von Fettaminen, welche abgeleitet sind von natürlichen Fettsäuren, wie N,N-Bis-(3-aminopropyl)dodecylamin und N,N-Bis-(3-aminopropyl)talgalkylamin, erhältlich als Triameen^{®} Y12D und Triameen^{®} YT (von Akzo Nobel);
- tertiäre Aminogruppen aufweisende Polyamine mit drei primären aliphatischen Aminogruppen, wie insbesondere Tris-(2-aminoethyl)amin, Tris-(2-aminopropyl)amin und Tris-(3-aminopropyl)amin;
- sekundäre Aminogruppen aufweisende Polyamine mit zwei primären aliphatischen Aminogruppen, wie insbesondere 3-(2-Aminoethyl)aminopropylamin, Bis-hexamethylentriamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis-(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin und N,N'-Bis-(3-amino-1-ethylpropyl)-2-methyl-1,5-pentandiamin.
- eine primäre und eine sekundäre Aminogruppe aufweisende Polyamine, wie insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, N-(2-Aminoethyl)piperazin, N-Methyl-1,3-propandiamin, N-Butyl-1,3-propandiamin, N-(2-Ethylhexyl)-1,3-propandiamin, N-Cyclohexyl-1,3-propandiamin, 3-Methylamino-1-pentylamin, 3-Ethylamino-1-pentylamin, 3-Cyclohexylamino-1-pentylamin, Fettdiamine wie N-Cocoalkyl-1,3-propandiamin und Produkte aus der Michael-artigen Additionsreaktion von primären aliphatischen Diaminen mit Acrylnitril, Malein- oder Fumarsäurediestern, Citraconsäurediestern, Acryl-und Methacrylsäureestern, Acryl- und Methacrylsäureamiden und Itaconsäurediestern, umgesetzt im Molverhältnis 1:1, weiterhin Produkte aus der partiellen reduktiven Alkylierung von primären aliphatischen Polyaminen mit Benzaldehyd oder ändern Aldehyden oder Ketonen, sowie partiell styrolisierte Polyamine wie Gaskamine^{®} 240 (von Mitsubishi Gas Chemical (MGC));
- aromatische Polyamine, wie insbesondere m- und p-Phenylendiamin, 4,4'-, 2,4' und 2,2'-Diaminodiphenylmethan, 3,3'-Dichloro-4,4'-diaminodiphenylmethan (MOCA), 2,4- und 2,6-Toluylendiamin, Mischungen von 3,5-Dimethylthio-2,4- und -2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink^{®} von Air Products), 1,2-Bis-(2-aminophenylthio)ethan, 2-Methylpropyl-(4-chloro-3,5-diaminobenzoat) und tert.Butyl-(4-chloro-3,5-diaminobenzoat);
- Addukte der genannten Polyamine mit Epoxiden und Epoxidharzen, insbesondere Addukte mit Diepoxiden im Molverhältnis von mindestens 2/1, Addukte mit Monoepoxiden im Molverhältnis von mindestens 1/1, sowie Umsetzungsprodukte aus Aminen und Epichlorhydrin, insbesondere jenes von 1,3-Bis-(aminomethyl)benzol, kommerziell erhältlich als Gaskamine^{®} 328 (von MGC);
- Polyamidoamine, welche Umsetzungsprodukte aus einer ein- oder mehrwertigen Carbonsäure, beziehungsweise deren Ester oder Anhydride, insbesondere einer Dimerfettsäure, und einem im stöchiometrischen Überschuss eingesetzten aliphatischen, cycloaliphatischen oder aromatischen Polyamin, insbesondere einem Polyalkylenamin wie beispielsweise DETA oder TETA, darstellen, insbesondere die kommerziell erhältlichen Polyamidoamine Versamid^{®} 100, 125, 140 und 150 (von Cognis), Aradur^{®} 223, 250 und 848 (von Huntsman), Euretek^{®} 3607 und 530 (von Huntsman) und Beckopox^{®} EH 651, EH 654, EH 655, EH 661 und EH 663 (von Cytec); und
- Phenalkamine, auch Mannich-Basen genannt, welche Umsetzungsprodukte einer Mannich-Reaktion von Phenolen, insbesondere Cardanol, mit Aldehyden, insbesondere Formaldehyd, und Polyaminen darstellen, insbesondere die kommerziell erhältlichen Phenalkamine Cardolite^{®} NC-541, NC-557, NC-558, NC-566, Lite 2001 und Lite 2002 (von Cardolite), Aradur^{®} 3440, 3441, 3442 und 3460 (von Huntsman) und Beckopox^{®} EH 614, EH 621, EH 624, EH 628 und EH 629 (von Cytec).

Bevorzugt als Polyamin A sind Polyamine ausgewählt aus der Gruppe bestehend aus 1,3-Pentandiamin (DAMP), 1,5-Diamino-2-methylpentan (MPMD), 2-Butyl-2-ethyl-1,5-pentandiamin (C11-Neodiamin), 1,6-Hexandiamin, 2,2,4- und 2,4,4-Trimethylhexamethylendiamin (TMD), 1,12-Dodecandiamin, 1,3-Diaminocyclohexan, Bis-(4-aminocyclohexyl)methan (H₁₂-MDA), Bis-(4-amino-3-methylcyclohexyl)methan, 1-Amino-3-aminomethyl-3,5,5-trimethylcyclohexan (IPDA), 1,3-Bis-(aminomethyl)cyclohexan, 1,3-Bis-(aminomethyl)benzol (MXDA), Bis-hexamethylentriamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (HEPA), Dipropylentriamin (DPTA), N-(2-Aminoethyl)-1,3-propandiamin (N3-Amin), N,N'-Bis(3-aminopropyl)ethylendiamin (N4-Amin), Polyoxyalkylen-Diamine und Polyoxyalkylen-Triamine mit einem Molekulargewicht im Bereich von 200 bis 500 g/mol, insbesondere die Typen Jeffamine^{®} D-230, Jeffamine^{®} D-400 und Jeffamine^{®} T-403, Polyanlidoamine, Phenalkamine, an primären Aminogruppen vollständig oder teilweise alkylierte Verbindungen der genannten Polyamine und Addukte der genannten Polyamine mit Epoxiden und Epoxidharzen. Diese bevorzugten Polyamine **A** sind besonders gut verträglich mit Epoxidharzen und ergeben Filme von sehr guter Qualität.

Besonders bevorzugt als Polyamin **A** sind mindestens eine sekundäre Aminogruppe aufweisende Polyamine, insbesondere Phenalkamine, Addukte von Polyaminen mit Epoxiden und Epoxidharzen, sowie primäre Polyamine, welche an mindestens einer primären Aminogruppe alkyliert sind, insbesondere styrolisiertes 1,3-Bis-(aminomethyl)benzol und benzylierte Polyalkylenamine, Diese bevorzugten Polyamine **A** neigen kaum zu Blushing-Effekten und ergeben mit Epoxidharzen auch unter feucht-kalten Bedingungen Filme von besonders hoher Qualität. Viele dieser Amine weisen aber eine hohe Viskosität auf, so dass sie ohne Verdünnung nur über eine ungenügende Verarbeitbarkeit als Epoxidharz-Beschichtung verfügen. Für emissionsarme Beschichtungen ist ihre Kombination mit dem Amin der Formel (I) besonders vorteilhaft, da dadurch niedrigviskose Härter zugänglich sind, die kaum Neigung zu Blushing aufweisen und zu Filmen von hoher Härte bei geringer Sprödigkeit aushärten, auch ohne die Anwesenheit von nicht einbaubaren Verdünnern.

Geeignet als Polyamin **A** sind insbesondere auch Mischungen umfassend mehrere der genannten Polyamine.

Am meisten bevorzugt als Polyamin **A** sind Polyamine, welche mit Epoxidharzen an sich gute Eigenschaften als Beschichtung aufweisen, aber eine eher hohe Viskosität aufweisen und aus Gründen der Verarbeitbarkeit und/oder zu hoher Sprödigkeit gerne mit nicht einbaubaren Verdünnern, wie beispielsweise Benzylalkohol, Alkylphenolen, styrolisiertem Phenol oder Kohlenwasserstoff-Harzen, versetzt würden, um den Anforderungen an hochwertige Beschichtungen zu genügen. Mit solchen nicht einbaubaren Verdünnern sind die Zusammensetzungen aber für Anwendungen, wo emissionsarme Systeme gefordert werden, nicht geeignet. Durch das Amin der Formel (I) hingegen, das bei der Aushärtung vollständig in die Harzmatrix eingebaut wird, entstehen nach der Aushärtung keine Emissionen,

Umfasst der Härter neben dem Amin der Formel (I) zusätzlich ein Polyamin **A,** so liegt das Verhältnis der Anzahl gegenüber Epoxidgruppen reaktiven Aminwasserstoffe des Amins der Formel (I) und der Anzahl gegenüber Epoxidgruppen reaktiven Aminwasserstoffe des Polyamins **A** typischerweise im Bereich von 0.05 bis 5, insbesondere 0.05 bis 2. Solche Härter weisen eine erniedrigte Viskosität auf und härten mit Epoxidharzen zu Filmen von geringer Sprödigkeit und einer breit einstellbaren Härte, von moderat bei höherer Einsatzmenge des Amins der Formel (I) bis hoch bei geringerer Einsatzmenge des Amins der Formel (I).

Besonders bevorzugt liegt dieses Verhältnis im Bereich von 0.1 bis 1. Solche Härter zeichnen sich durch eine niedrige Viskosität und eine schnelle Aushärtung mit Epoxidharzen zu Filmen von hoher Härte bei geringer Sprödigkeit aus.

Im beschriebenen Härter liegt das Gewichtsverhältnis des Amins der Formel (I) und des Polyamins **A** bevorzugt im Bereich von 0.2 bis 2, insbesondere im Bereich von 0.4 bis 1.5. Solche Härter zeichnen sich durch eine niedrige Viskosität und eine schnelle Aushärtung mit Epoxidharzen zu Filmen von hoher Härte bei geringer Sprödigkeit aus.

Der erfindungsgemässe Härter kann zusätzlich zum Amin der Formel (I) mindestens einen Beschleuniger umfassen.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; weiterhin tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo-[5.4.0]undec-7-en, Guanidine wie insbesondere 1,1,3,3-Tetramethylguanidin, Phenole, insbesondere Bisphenole, Phenol-Harze und Mannich-Basen wie insbesondere 2-(Dimethylaminomethyl)phenol, 2,4,6-Tris-(dimethylaminomethyl)phenol und Polymere aus Phenol, Formaldehyd und N,N-Dimethyl-1,3-propandiamin, Phosphite wie insbesondere Di- und Triphenylphosphite, sowie Mercaptogruppen aufweisende Verbindungen, wie sie bereits vorgängig genannt wurden.

Bevorzugte Beschleuniger sind Salicylsäure und 2,4,6-Tris-(dimethylaminomethyl)phenol.

Der erfindungsgemässe Härter kann zusätzlich zum Amin der Formel (I) weiterhin mindestens einen nicht einbaubaren Verdünner umfassen, wie insbesondere Xylol, 2-Methoxyethanol, Dimethoxyethanol, 2-Ethoxyethanol, 2-Propoxyethanol, 2-lsopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykolmonoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, Adipate, Sebacate, Phthalate, Benzoate, organische Phosphor- und Sulfonsäureester und Sulfonamide. Bevorzugt sind Benzylalkohol, Dodecylphenol, tert.Butylphenol, styrolisiertes Phenol und phenolgruppenhaltige aromatische Kohlenwasserstoffharze, insbesondere die Novares^{®}-Typen LS 500, LX 200, LA 300 und LA 700 (von Rütgers).

Bevorzugt enthält der Härter keinen oder nur einen geringen Gehalt an nicht einbaubaren Verdünnern, besonders bevorzugt weniger als 25 Gewichts-%, insbesondere weniger als 10 Gewichts-% und am meisten bevorzugt weniger als 5 Gewichts-%. Insbesondere werden dem Härter keine nicht einbaubaren Verdünner zugesetzt.

Weiterhin kann der beschriebene Härter weitere gegenüber Epoxidgruppen reaktive Substanzen enthalten, wie insbesondere
- Monoamine, wie insbesondere Benzylamin, Cyclohexylamin, 2-Phenylethylamin, 2-Methoxyphenylethylamin, 4-Methoxyphenylethylamin, 3,4-Dimethoxyphenylethylamin (Homoveratrylamin), 1- und 2-Butylamin, Isobutylamin, tert.-Butylamin, 3-Methyl-2-butylamin, 1-Hexylamin, 1-Octylamin, 2-Ethyl-1-hexylamin, 2-Methoxy-1-ethylamin, 2-Ethoxy-1-ethylamin, 3-Methoxy-1-propylamin, 3-Ethoxy-1-propylamin, 3-(2-Ethylhexyloxy)propylamin, 3-(2-Methoxyethoxy)propylamin;
- sekundäre aliphatische Polyamine, wie insbesondere N,N'-Dibutyl-ethylendiamin, N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N⁴-Cyclohexyl-2-methyl-N²-(2-methylpropyl)-2,4-pentandiamin, N,N'-Dialkyl-1,3-xylylendiamin, Bis-(4-(N-3-butylamino)-cyclohexyl)-methan (Clearlink^{®} 1000 von UOP), N-alkylierte Polyetheramine, beispielsweise die Jeffamine^{®}-Typen SD-231, SD-401, ST-404 und SD-2001 (von Huntsman), Produkte aus der Michael-artigen Additionsreaktion von primären aliphatischen Polyaminen mit Michaelakzeptoren wie Acrylnitril, Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd, sowie weiterhin Produkte aus der reduktiven Alkylierung von primären aliphatischen Polyaminen mit Benzaldehyd oder andern Aldehyden oder Ketonen;
- flüssige Mercaptan-terminierte Polysulfid-Polymere, bekannt unter dem Markennamen Thiokol^{®} (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 und LP-2; sowie weiterhin bekannt unter dem Markennamen Thioplast^{®} (von Akzo Nobel), insbesondere die Typen G 10, G 112, G 131, G 1, G 12, G 21, G 22, G 44 und G 4;
- Mercaptan-terminierte Polyoxyalkylen-Ether, erhältlich beispielsweise durch Umsetzung von Polyoxyalkylendi- und -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure^{®} (von Cognis), insbesondere die Typen WR-8, LOF und 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat), sowie die Veresterungsprodukte von Polyoxyalkylendiolen und -triolen, ethoxyliertem Trimethylolpropan und Polyester-Diolen mit Thiocarbonsäuren wie Thioglykolsäure und 2- oder 3-Mercaptopropionsäure; und
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) und Ethandithiol.

Ein weiterer Gegenstand der Erfindung ist eine Epoxidharz-Zusammensetzung, enthaltend
a) mindestens ein Epoxidharz, und
b) mindestens einen Härter umfassend das Amin der Formel (I), wie vorgängig beschrieben.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.

Als Epoxidharz besonders geeignet sind sogenannte Polyepoxid-Flüssigharze, im folgenden als "Flüssigharz" bezeichnet. Diese weisen eine Glasübergangstemperatur auf, welche üblicherweise unterhalb von 25 °C liegt, im Gegensatz zu den sogenannten Festharzen, welche eine Glasübergangstemperatur oberhalb von 25 °C aufweisen und sich zu bei 25 °C schüttfähigen Pulvern zerkleinern lassen.

In einer Ausführungsform handelt es sich beim Flüssigharz um ein aromatisches Polyepoxid. Dafür geeignet sind beispielsweise Flüssigharze der Formel (II), 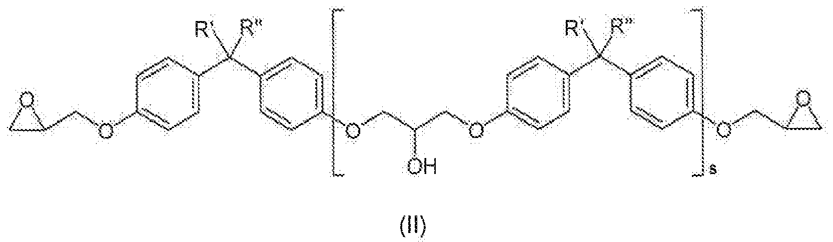 wobei R' und R" unabhängig voneinander jeweils für ein Wasserstoffatom oder für eine Methylgruppe stehen, und s im Mittel für einen Wert von 0 bis 1 steht. Bevorzugt sind solche Flüssigharze der Formel (II), bei denen der Index s im Mittel für einen Wert von kleiner als 0.2 steht.

Bei den Flüssigharzen der Formel (II) handelt es sich um Diglycidylether von Bisphenol-A, Bisphenol-F und Bisphenol-A/F, wobei A für Aceton und F für Formaldehyd steht, welche als Edukte zur Herstellung dieser Bisphenole dienen. Ein Bisphenol-A-Flüssigharz weist dementsprechend Methylgruppen, ein Bisphenol-F-Flüssigharz Wasserstoffatome und ein Bisphenol-A/F-Flüssigharz sowohl Methylgruppen als auch Wasserstoffatome als R' und R" in Formel (II) auf. Im Fall von Bisphenol-F können auch Stellungsisomere vorhanden sein, insbesondere abgeleitet von 2,4'- und 2,2'-Hydroxyphenylmethan.

Weitere geeignete aromatische Flüssigharze sind die Glycidylisierungsprodukte von
- Dihydroxybenzol-Derivaten wie Resorcin, Hydrochinon und Brenzkatechin;
- weiteren Bisphenolen oder Polyphenolen wie Bis-(4-hydroxy-3-methylphenyl)-methan, 2,2-Bis-(4-hydroxy-3-methylyphenyl)-propan (Bisphenol-C), Bis-(3,5-dimethyl-4-hydroxyphenyl)-methan, 2,2-Bis-(3,5-dimethyl-4-hydroxyphenyl)-propan, 2,2-Bis-(3,5-dibromo-4-hydroxyphenyl)-propan, 2,2-Bis-(4-hydroxy-3-tert.-butylphenyl)-propan, 2,2-Bis-(4-hydroxyphenyl)-butan (Bisphenol-B), 3,3-Bis-(4-hydroxyphenyl)-pentan, 3,4-Bis-(4-hydroxyphenyl)-hexan, 4,4-Bis-(4-hydroxyphenyl)-heptan, 2,4-Bis-(4-hydroxyphenyl)-2-methylbutan, 2,4-Bis-(3,5-dimethyl-4-hydroxyphenyl)-2-methylbutan, 1,1-Bis-(4-hydroxyphenyl)-cyclohexan (Bisphenol-Z), 1,1-Bis-(4-hydroxyphenyl)-3,3,5-trimethylcyclohexan (Bisphenol-TMC), 1,1-Bis-(4-hydroxyphenyl)-1-phenylethan, 1,4-Bis[2-(4-hydroxyphenyl)-2-propyl]-benzol) (Bisphenol-P), 1,3-Bis-[2-(4-hydroxyphenyl)-2-propyl]-benzol) (Bisphenol-M), 4,4'-Dihydroxydiphenyl (DOD), 4,4'-Dihydroxybenzophenon, Bis-(2-hydroxynaphth-1-yl)-methan, Bis-(4-hydroxynaphth-1-yl)-methan 1,5-Dihydroxy-naphthalin, Tris-(4-hydroxyphenyl)-methan, 1,1,2,2-Tetrakis-(4-hydroxyphenyl)-ethan, Bis-(4-hydroxyphenyl)-ether, Bis-(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin (MDA), 4,4'-Methylendiphenyldi-(N-methyl)-amin, 4,4'-[1,4-Phenylen-bis-(1-methyl-ethyliden)]-bisanilin (Bisanilin-P), 4,4'-[1,3-Phenylen-bis-(1-methyl-ethyliden)]-bisanilin (Bisanilin-M).

Als Epoxidharz eignet sich auch ein aliphatisches oder cycloaliphatisches Polyepoxid, wie beispielsweise
- ein Glycidylether eines gesättigten oder ungesättigten, verzweigten oder unverzweigten, zyklischen oder offenkettigen C₂- bis C₃₀-Diols, wie beispielsweise Ethylenglykol, Propylenglykol, Butylenglykol, Hexandiol, Octandiol, ein Polypropylenglykol, Dimethylolcyclohexan, Neopentylglykol oder Dibromo-neopentylglykol;
- ein Glycidylether eines tri- oder tetrafunktionellen, gesättigten oder ungesättigten, verzweigten oder unverzweigten, zyklischen oder offenkettigen Polyols wie Rizinusöl, Trimethylolpropan, Trimethylolethan, Pentaerythrol, Sorbit oder Glycerin, sowie alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat und Triglycidylisocyanurat, sowie Umsetzungsprodukte von Epichlorhydrin und Hydantoin.

Als Epoxidharz möglich sind auch ein Bisphenol-A-, -F- oder -A/F-Festharz, welches ähnlich aufgebaut ist wie die bereits genannten Flüssigharze der Formel (II), aber anstelle des Index s einen Wert von 2 bis 12 aufweist, und eine Glasübergangstemperatur oberhalb von 25 °C aufweist.

Als Epoxidharz eignen sich schliesslichauch Epoxidharze aus der Oxidation von Olefinen, beispielsweise aus der Oxidation von Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz bevorzugt sind Flüssigharze auf der Basis eines Bisphenols, insbesondere auf der Basis von Bisphenol-A, Bisphenol-F- oder Bisphenol-A/F, wie sie kommerziell beispielsweise von Dow, Huntsman und Hexion erhältlich sind. Diese Flüssigharze weisen eine für Epoxidharze niedrige Viskosität und im ausgehärteten Zustand gute Eigenschaften als Beschichtungen auf. Sie können gegebenenfalls in Kombination mit Bisphenol A-Festharz oder Bisphenol-F-Novolak-Epoxidharz vorhanden sein.

Das Epoxidharz kann einen Reaktivverdünner, insbesondere einen mindestens eine Epoxidgruppe aufweisenden Reaktivverdünner, enthalten. Als Reaktivverdünner geeignet sind beispielsweise die Glycidylether von ein- oder mehrwertigen Phenolen und aliphatischen oder cycloaliphatischen Alkoholen, wie insbesondere die bereits genannten Polyglycidylether von Die- oder Polyolen, sowie weiterhin insbesondere Phenylglycidylether, Kresylglycidylether, p-n-Butyl-phenylglycidylether, p-tert.Butyl-phenylglycidylether, Nonylphenylglycidylether, Allylglycidylether, Butylglycidylether, Hexylglycidylether, 2-Ethylhexylglycidylether, sowie Glycidylether von natürlichen Alkoholen, wie zum Beispiel C₈- bis C₁₀-Alkylglycidylether oder C₁₂₋ bis C₁₄-Alkylglycidylether, Die Zugabe eines Reaktivverdünners zum Epoxidharz bewirkt eine Reduktion der Viskosität, sowie - im ausgehärteten Zustand der Epoxidharz-Zusammensetzung - eine Reduktion der Glasübergangstemperatur und der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate und Reaktivgruppen-aufweisende Silikone;
- Polymere, wie beispielsweise Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere chlorsulfonierte Polyethylene und Fluor-haltige Polymere, Sulfonamidmodifizierte Melamine und gereinigte Montan-Wachse;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln:
- Fasern, insbesondere Glasfasern, Kohlefasern, Metallfasern, Keramikfasern oder Kunststofffasern wie Polyamidfasern oder Polyethylenfasern,
- Pigmente, beispielsweise Titandioxid und Eisenoxide;
- die vorgenannten Beschleuniger;
- Rheologie-Modifizierer, wie insbesondere Verdickungsmittel, zum Beispiel Schichtsilikate wie Bentonite, Derivate von Rizinusöl, hydriertes Rizinusöl, Polyamide, Polyurethane, Harnstoffverbindungen, pyrogene Kieselsäuren, Celluloseether und hydrophob modifizierte Polyoxyethylene;
- Haftverbesserer, beispielsweise Organoalkoxysilane wie Aminosilane, Mercaptosilane, Epoxysilane, Vinylsilane, (Meth)acrylsilane, Isocyanatosilane, Carbamatosilane, Alkylsilane, S-(Alkylcarbonyl)-mercaptosilane und Aldiminosilane, sowie oligomere Formen dieser Silane, insbesondere 3-Glycidoxypropyltrimethoxysilan, 3-Aminopropyltrimethoxysilan, N-(2-Aminoethyl)-3-aminopropyl-trimethoxysilan, N-(2-Aminoethyl)-N'-[3-(trimethoxysilyl)propyl]ethylendiamin, 3-Mercaptopropyltrimethoxysilan, 3-Isocyanatopropyltrimethoxysilan, 3-Ureidopropyltrimethoxysilan, 3-Chloropropyltrimethoxysilan, Vinyltrimethoxysilan, oder die entsprechenden Organosilane mit Ethoxygruppen anstelle der Methoxygruppen;
- Stabilisatoren gegen Oxidation, Wärme, Licht und UV-Strahlung;
- flammhemmende Substanzen, insbesondere Verbindungen wie Aluminiumhydroxid (Al(OH)₃; auch ATH für "Aluminiumtrihydrat" genannt), Magnesiumhydroxid (Mg(OH)₂: auch MDH für "Magnesiumdihydrat" genannt), Ammoniumsulfat ((NH₄)₂SO₄), Borsäure (B(OH)₃), Zinkborat, Zinkphosphat, Melaminborat und Melamincyanurat; Phosphor-haltige Verbindungen wie Ammoniumphosphat ((NH₄)₃PO4), Ammoniumpolyphosphat, Melaminphosphat, Melaminpyrophosphat, Triphenylphosphat, Diphenylkresylphosphat, Trikresylphosphat, Triethylphosphat, Tris-(2-ethylhexyl)phosphat, Trioctylphosphat, Mono-, Bis- und Tris-(isopropylphenyl)phosphat, Resorcinol-bis(diphenylphosphat), Resorcinol-diphosphat-Oligomer, Tetraphenyl-resorcinol-diphosphit, Ethylendiamin-diphosphat und Bisphenol-A-bis(diphenylphosphat); Halogen-haltige Verbindungen wie Chloroalkylphosphate, insbesondere Tris-(chloroethyl)phosphat, Tris-(chloropropyl)phosphat und Tris-(dichloroisopropyl)phosphat, polybromierte Diphenylether, insbesondere Decabromdiphenylether, polybromiertes Diphenyloxid, Tris-[3-Bromo-2,2-bis(bromomethyl)-propyl]phosphat, Tetrabromo-Bisphenol-A, Bis-(2,3-dibromopropylether) von Bisphenol A, bromierte Epoxidharze, Ethylen-bis(tetrabromophtalimid), Ethylen-bis(dibromonorbornandicarboximid), 1,2-Bis-(tribromophenoxy)ethan, Tris-(2,3-dibromopropyl)isocyanurat, Tribromophenol, Hexabromocyclododecan, Bis-(hexachlorocyclopentadieno)cyclooctan und Chlorparaffine; sowie Kombinationen aus einer Halogen-haltigen Verbindung und Antimontrioxid (Sb₂O₃) oder Antimonpentoxid (Sb₂O₅);
- oberflächenaktiven Substanzen, wie insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und Beschleuniger, insbesondere Salicylsäure oder 2,4,6-Tris-(dimethylaminomethyl)phenol.

Bevorzugt enthält die Epoxidharz-Zusammensetzung keinen oder nur einen geringen Gehalt an nicht einbaubaren Verdünnern, besonders bevorzugt weniger als 10 Gewichts-%, insbesondere weniger als 5 Gewichts-%, am meisten bevorzugt weniger als 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen im Bereich von 0.5 bis 1.5, bevorzugt 0.7 bis 1.2.

Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Insbesondere ist die Epoxidharz-Zusammensetzung eine zweikomponentige Zusammensetzung, bestehend aus
(i) einer Harz-Komponente enthaltend mindestens ein Epoxidharz und
(ii) einer Härter-Komponente enthaltend den Härter umfassend das Amin der Formel (I), wie vorgängig beschrieben.

Die Komponenten der zweikomponentigen Zusammensetzung werden jeweils in einem eigenen Gebinde gelagert. Weitere Bestandteile der zweikomponentigen Epoxidharz-Zusammensetzung können als Bestandteil der Harz-oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern.

Zur Anwendung derzweikomponentigen Epoxidharz-Zusammensetzung werden die Harz-Komponente und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.

Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50 °C, bevorzugt bei etwa 10 bis 30 °C, liegt.

Mit der Vermischung der beiden Komponenten beginnt die Aushärtung durch chemische Reaktion, wie vorgängig beschrieben. Die Aushärtung erfolgt insbesondere bei Umgebungstemperatur, welche typischerweise im Bereich von etwa 5 bis 50 °C, bevorzugt bei etwa 10 bis 30 °C, liegt, Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.

Ein weiterer Gegenstand der Erfindung ist somit auch eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips und Natursteine wie Granit oder Marmor;
- Metalle und Legierungen, wie Aluminium, Eisen, Stahl und Buntmetalle, sowie oberflächenveredelte Metalle und Legierungen, wie verzinkte oder verchromte Metalle;
- Leder, Textilien, Papier, Holz, mit Harzen, beispielsweise Phenol-, Melamin-oder Epoxidharzen, gebundene Holzwerkstofie, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Polyester, Epoxidharze, Polyurethane (PUR), Polyoxymethylen (POM), Polyolefine (PO), Polyethylen (PE) oder Polypropylen (PP), Ethylen/Propylen-Copolymere (EPM) und Ethylen/Propylen/Dien-Terpolymere (EPDM), wobei die Kunststoffe bevorzugt mittels Plasma, Corona oder Flammen oberflächenbehandelt sein können;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- beschichtete Substrate, wie pulverbeschichtete Metalle oder Legierungen;
- Farben und Lacke, insbesondere Automobildecklacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten oder dergleichen, wobei dabei entstehende Stäube vorteilhaft abgesaugt werden, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundwerkstoff (Composite), Vergussmasse, Dichtstoff, Klebstoff, Belag, Beschichtung, Anstrich, Lack, Versiegelung, Grundierung, Primer, Schaum, Formblock, Elastomer, Faser, Folie oder Membran.

Insbesondere verwendbar ist sie als Vergussmasse, Dichtstoff und Klebstofl, wie beispielsweise als Elektrovergussmasse, Abdichtungsmasse, Spachtelmasse, Fugendichtstofl, Montageklebstofi, Karrosserieklebstoff, Sandwichelementklebstoff, Halbschalenklebstoff, beispielsweise für Rotorblätter von Windkraftanlagen, Brückenelementklebstofi, Kaschierklebstoff, Laminierklebstofi oder Verankerungsklebstoff. sowie weiterhin als Belag, Beschichtung, Anstrich, Lack, Versiegelung, Grundierung und Primer für Bau- und Industrieanwendungen, wie insbesondere als Bodenbelag und Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle oder Kunststoffe, beispielsweise zur Oberflächenversiegelung von Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden, wobei diese Beschichtungen die jeweiligen Substrate insbesondere gegen Korrosion, Abrasion, Feuchtigkeit, Wasser- und/oder Salzeinwirkung oder Chemikalien schützen; sowie weiterhin als Voranstrich, Haftanstrich, Korrosionsschutz-Primer oder zur Hydrophobierung von Oberflächen. Insbesondere geeignet ist die beschriebene Zusammensetzung auch als Beschichtung für sogenannt schweren Korrosionsschutz im und am Wasser, insbesondere auch im und am Meerwasser. Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft verwendbar ist die beschriebene Epoxidharz-Zusammensetzung als Beschichtung. Als Beschichtung werden dabei flächig aufgebrachte Beläge aller Art verstanden, insbesondere auch Anstriche, Lacke, Versiegelungen, Grundierungen und Primer, wie vorgängig beschrieben, Insbesondere vorteilhaft verwendbar ist die beschriebene Epoxidharz-Zusammensetzung in emissionsarmen Systemen mit Öko-Gütesiegeln, beispielsweise nach Emicode (EC1 Plus), AgBB, DIBT, Der Blaue Engel, AFSSET, RTS (M1) und US Green Building Council (LEED).

Ein weiterer Gegenstand der Erfindung ist somit die Verwendung der beschriebenen Epoxidharz-Zusammensetzung als Beschichtung

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende Beschichtung auf überwiegend ebene Flächen oder als Anstrich appliziert werden kann. Bevorzugt weist die Epoxidharz-Zusammensetzung bei dieser Applikation unmittelbar nach dem Vermischen der Harz- und der Härter-Komponente eine Viskosität, gemessen bei 20 °C, im Bereich von 300 bis 2'000 mPa·s, bevorzugt im Bereich von 300 bis 1'500 mPa·s, insbesondere im Bereich von 300 bis 1 '000 mPa·s auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt beispielsweise durch Aufgiessen auf das zu beschichtende Substrat. Dabei wird die Zusammensetzung im flüssigen Zustand mit Hilfe beispielsweise eines Rakels oder einer Zahntraufel gleichmässig verteilt. Zusätzlich kann die verteilte Zusammensetzung mit einer Stachelwalze egalisiert und entlüftet werden. Die Applikation kann aber auch manuell durch Pinsel oder Roller oder als Spritzapplikation erfolgen, beispielsweise als Korrosionsschutzbeschichtung auf Stahl. Bei der Aushärtung entstehen typischerweise weitgehend klare, glänzende und nichtklebrige Filme von geringer Sprödigkeit, welche eine gute Haftung zu verschiedensten Substraten aufweisen und über eine hohe Härte verfügen. Aufgrund des Amins der Formel (I) im Härter sind selbstverlaufende Epoxidharz-Beschichtungen zugänglich, welche ohne oder mit nur geringen Anteilen an nicht einbaubaren Verdünnern auskommen, und der Gehalt an primären Aminogruppen kann in der Beschichtung so tief gehalten werden, dass kaum Reaktionen mit CO₂ aus der Luft auftreten. Dadurch bleiben Blushing-Effekte bei der flächigen Anwendung auch unter ungünstigen, das heisst das Blushing begünstigenden, Reaktionsbedingungen, insbesondere bei tiefer Aushärtungstemperaturen im Bereich von 5 bis 10 °C und hoher Luftfeuchtigkeit, weitgehend aus.

Ein weiterer Gegenstand der Erfndung ist ein Artikel, erhalten aus der Verwendung der beschriebenen Epoxidharz-Zusammensetzung als Beschichtung.

Die beschriebene Epoxidharz-Zusammensetzung zeichnet sich durch zahlreiche vorteilhafte Eigenschaften aus. Sie weist nur wenig Geruch auf und ist bei Raumtemperatur überraschend dünnflüssig, womit sie auch ohne weitere Verdünner gut verarbeitbar ist, insbesondere auch bei flächiger Anwendung. Sie härtet bei Umgebungstemperatur und insbesondere auch bei feucht-kalten Bedingungen überraschend schnell und ohne störende Blushing-Effekte aus und verfügt im ausgehärteten Zustand über eine überraschend hohe Härte bei geringer Sprödigkeit. Dadurch ist sie in der Lage, mechanischer Beanspruchung gut zu widerstehen, was besonders in der Anwendung als Schutzbeschichtung oder als Bodenbelag besonders wichtig ist. Ausgehärtete Filme sind typischerweise nicht trüb und weisen eine ebenmässig glänzende, kraterfreie und nichtklebrige Oberfläche auf.

Besonders vorteilhafte Eigenschaften weisen Epoxidharz-Zusammensetzungen auf, welche neben dem Amin der Formel (I) noch mindestens ein Polyamin **A** umfassen. Diese Epoxidharz-Zusammensetzungen zeigen eine überraschende Kombination von Vorteilen: Einerseits übt das Amin der Formel (I) eine ausgezeichnete verdünnende Wirkung auf die Zusammensetzung aus, wodurch die Zusammensetzung unmittelbar nach dem Vermischen der Komponenten eine niedrige Viskosität aufweist, und andererseits härtet die Zusammensetzung schnell aus. Weiterhin verringert das Amin der Formel (I) die Neigung zu Blushing-Effekten, und schliesslich härtet die Zusammensetzung zu Filmen von ausgezeichneter Qualität bezüglich Klarheit, Glanz, Oberflächenklebrigkeit, hoher Härte bei geringer Sprödigkeit. Aufgrund der Tatsache, dass das Amin der Formel (I) keine primären Aminogruppen und lediglich zwei sekundäre Aminogruppen enthält, sind die schnelle Aushärtung, die erreichbaren hohen Härten und die Nichtklebrigkeit besonders überraschend.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

### 1. Beschreibung der Messmethoden

Der **Amingehalt,** das heisst der totale Gehalt an Aminogruppen in den hergestellten Verbindungen, wurde titrimetrisch bestimmt (mit 0.1N HClO₄ in Eisessig, gegen Kristallviolett) und ist stets angegeben in mmol N/g.
**Infrarotspektren** wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).
**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS).
**Massenspektren** (FIMS) wurden auf einem hochauflösenden Massenspektrometer des Typs Thermo Scientific LTQ Orbitrap XL gemessen, indem 500 µl der in Methanol gelösten Probe (100 µg/ml) bei einer Injektionsrate von 10 µl/min. und einer Flussrate des Trägers (1 mM Ammoniumformiat in Methanol) von 500 µl/min direkt in den Mas-senspektrometer eingespritzt wurden; die Detektion erfolgte mittels Elektrospray-lonisation (ESI⁺).

Die **Viskosität** (η) wurde bei 20 °C auf einem thermostatisierten Kegel-Platten-Viskosimeter Rheotec RC30 (Kegeldurchmesser 50 mm, Kegelwinkel 1°, Kegelspitze-Piatten-Abstand 0.05 mm, Scherrate 10-100 s⁻¹) gemessen.

### 2. verwendete Substanzen:

| | |
|---|---|
| EP-Addukt 1: | Umsetzungsprodukt aus 116.0 GT 1,5-Diamino-2-methyl-pentan und 182 GT Araldite^{®} DY-K; NH-Equivalent = 99.3 g/Eq; η = 5'830 mPa·s |
| EP-Addukt 2: | Umsetzungsprodukt aus 136.2 Gewichtsteilen (GT) 1,3-Bis-(aminomethyl)benzol und 182 GT Araldite^{®} DY-K; NH-Equivalent = 106.1 g/Eq; η = 59'490 mPa·s |
| Gaskamine^{®} A-229 (MGC) | Reaktionsprodukt von 1,3-Bis-(aminomethyl)benzol mit Acrylnitril; NH-Equivalent = 102 g/Eq; η = 230 mPa·s |
| Gaskamine^{®} 240 (MGC) | Styrelisiertes 1,3-Bis-(aminomethyl)benzol; NH-Equivalent = 103 g/Eq: η = 165 mPa·s |
| Gaskamine^{®} 328 (MGC) | Reaktionsprodukt von 1,3-Bis-(aminomethyl) benzol mit Epichlorhydrin; NH-Equivalent = 55 g/Eq: η = 12'720 mPa·s |
| Polypox^{®} IH 7011 (UPPC Dow) | mod. Polyamin, Härter für emissionsarme EP-Systeme; NH-Equivalent = 82 g/Eq; η = 810 mPa·s |
| Aradur^{®} 3442 (Huntsman) | Phenalkamin; NH-Equivalent = 125 g/Eq; η = 10'210 mPa·s |
| Araldite^{®} DY-K (Huntsman) | Monoglycidylether von Kresol; EEW ca. 182 g/Eq |
| Araldite^{®} GY 250 (Huntsman) | Bisphenol-A-Diglycidylether; Epoxy-Equivaient ca, 187.5 g/Eq |
| Araldite^{®} DY-E (Huntsman) | Monoglycidylether eines C₁₂- bis C₁₄-Alkohols; Epoxy-Equivalent ca. 290 g/Eq |
| Ancamine^{®} K 54 (Air Products) | 2,4,6-Tris-(dimethylaminomethyl)phenol |

### 3. Herstellung von Aminen

### Amin 1: N,N'-Dibenzyl-m-xylylendiamin

In einem Rundkolben wurden 21.2 g Benzaldehyd und 13.6 g 1,3-Bis-(aminomethyl)benzol unter Stickstoffatmosphäre in ausreichend Isopropanol gelöst. Die Lösung wurde während 30 Minuten bei Raumtemperatur gerührt und anschliessend bei einem Wasserstoff-Druck von 80 bar, einer Temperatur von 80 °C und einem Fluss von 3 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die Lösung im Vakuum bei 80 °C eingeengt. Erhalten wurde ein klares, gelbliches Öl mit einer Viskosität von 230 mPa·s bei 20 °C, einem Amingehalt von 6.41 mmol N/g und einer Reinheit von 94.4 % (bestimmt mittels Gas-Chromatographie).
FT-IR: 3024, 2910, 2810, 1604, 1494, 1452, 1108, 1028, 784, 732, 694.
¹H-NMR (CDCl₃, 300 K): δ 7.4 - 7.1 (*m*, 14 H, Ar-H), 3.77 (s, 8 H, CH₂), 1.70 (s, 2 H, NH).
FILMS: m/z = 317.20203 ([MH⁺]; theoretische Masse für C₂₂H₂₅N₂: 317.20123).

### Amin 2: N-Benzyl-3-(N-benzyl)aminomethyl-3,5,5-trimethylcyclohexylamin

Auf die gleiche Weise wie für Beispiel 1 beschrieben wurden 21.2 g Benzaldehyd und 17.0 g 1-Amino-3-aminomethyl-3,5,5-trimethylcyclohexan (= Isophorondiamin) umgesetzt. Erhalten wurde ein klares, farbloses Öl mit einer Viskosität von 590 mPa·s bei 20 °C und einem Amingehalt von 5.82 mmol N/g.

### Amin 3: N,N'-D)benzyl-2-methylpentan-1,5-diamin

Auf die gleiche Weise wie für Beispiel 1 beschrieben wurden 21.2 g Benzaldehyd und 11.6 g 1,5-Diamino-2-methylpentan umgesetzt. Erhalten wurde ein klares, gelbliches Öl mit einer Viskosität von 420 mPa·s bei 20 °C und einem Amingehalt von 6.67 mmol N/g.

### 4. Herstellung von Härtern

### Beispiel 1 bis 5

Für jedes Beispiel wurde der in der Tabelle 1 angegebene Härter nach dem Stand der Technik mit dem Amin **1** in den angegebenen Mengen (in Gewichtsteilen) mittels eines Zentrifugalmischers (SpeedMixer™ DAC 150, FlackTek Inc.) vermischt. 1 Stunde nach dem Vermischen wurde jeweils die Viskosität des erhaltenen Härters bestimmt. (Das Verhältnis zwischen der Anzahl NH-Equivalente des Amins **1** und der Anzahl NH-Equivalente des Härters nach dem Stand der Technik betrug jeweils 0.33.)

**Tabelle 1: Zusammensetzungen und Viskositäten der Härter aus den Beispielen 1 bis 5, gemessen bei 20 °C.**

| **Beispiel** | **1** | **2** | **3** | **4** | **5** |
|---|---|---|---|---|---|
| EP-Addukt 1 | 74.5 | - | - | - | - |
| EP-Addukt 2 | - | 79.6 | - | - | - |
| Gaskamine^{®} 328 | - | - | 41.3 | - | - |
| Polypox^{®} IH 7011 | - | - | - | 61.5 | - |
| Aradur^{®} 3442 | - | - | - | - | 93.8 |
| Amin 1 | 39.6 | 39.6 | 39.6 | 39.6 | 39.6 |
| Viskosität ohne Amin 1 [mPa·s] | 5'830 | 59'490 | 12'720 | 810 | 10'210 |
| Viskosität mit Amin 1 [mPa·s] | 1'710 | 5'070 | 1'600 | 420 | 1'730 |

### 5. Herstellung von Epoxidharz-Zusammensetzungen

### Beispiele und Vergleichsbeispiele 6 bis 17

Für jedes Beispiel wurden die in den Tabellen 2 bis 4 angegebenen Inhaltsstoffe in den angegebenen Mengen (in Gewichtsteilen) mittels eines Zentrifugalmischers (SpeedMixer™ DAC 150, FlackTek Inc.) vermischt. 10 Minuten nach dem Vermischen wurde jeweils die Viskosität der Zusammensetzungen bestimmt ("Viskosität (10')"). Weiterhin wurde jeweils ein erster Film in einer Schichtdicke von 500 µm auf eine Glasplatte aufgezogen und dieser bei 23±1 °C und 50±5 % relativer Feuchtigkeit (= Normklima, im folgenden abgekürzt mit "NK") gelagert, beziehungsweise ausgehärtet. Nach 4 Wochen wurde der Aspekt der Filme beurteilt (in den Tabellen mit "Aspekt (NK)" bezeichnet). Als "schön" wurde ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet. Weiterhin wurde die Königshärte (Pendelhärte nach König, gemessen nach DIN EN ISO 1522) der Filme nach 2 Tagen ("Königshärte (NK) (2d)") bzw. nach 4 Tagen ("Königshärte (NK) (4d)") bzw. nach 7 Tagen ("Königshärte (NK) (7d)") bzw. nach 4 Wochen ("Königshärte (NK) (4w)") bestimmt. Weiterhin wurde jeweils ein weiter Film in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 4 Wochen im NK gelagert, beziehungsweise ausgehärtet. 24 Stunden nach der Applikation wurde auf den Film ein Flaschendeckel aus Polypropylen aufgesetzt, unter weichem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieser Filme beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/oder den aufgesetzten Deckel sichtbar waren. Trat an der Markierung eine Verfärbung oder Trübung auf, so ist dies ebenfalls angegeben. An den so ausgehärteten Filmen wurde wiederum die Königshärte bestimmt, jeweils nach 7 Tagen bei 8 °C und 80 % relativer Feuchtigkeit ("Königsh. (8°/80%) (7d kalt)"), dann nach weiteren 2 Tagen im NK ("Königsh. (8°/80%) (+2d NK)") bzw. 7 Tagen im NK ("Königsh. (8°/80%) (+7d NK)") bzw. 4 Wochen im NK ("Königsh. (8°/80%) (+4w NK)"). An den im Normklima gehärteten ersten Filmen wurde nach 2 Monaten als Mass für die Sprödigkeit die Neigung zum Splittern (in den Tabellen als "Splitter-Neigung" bezeichnet) bestimmt, indem mit einer Ahle durch schräges Andrücken in einem Winkel von etwa 45° vom Film Späne abgelöst wurden. Gelang dies ohne jegliches Wegsplittern von Bruchstücken des Films, wurde die Splitter-Neigung als "kleine" beurteilt. Ansonsten wurde sie mit "schwach", "mittels" oder"stark" bezeichnet, je nachdem, wie leicht vom Film beim Druck der Messerspitze zum Abhobeln eines Spans Bruchstücke absplitterten.

Die Resultate sind in den Tabellen 2 bis 4 angegeben.

**Tabelle 2: Zusammensetzung und Eigenschaften des Beispiels 6 und der Vergleichsbeispiele 7 bis 10. "n.m," steht für "nicht messbar", da der Film klebrig war; "Königsh." steht für "Königshärte";**

| **Beispiel** | | **6** | **7 (Vergleich)** | **8 (Vergleich)** | **9 (Vergleich)** | **10 (Vergleich)** |
|---|---|---|---|---|---|---|
| Araldite^{®} GY-250 | | 167.2 | 167.2 | 167.2 | 167.2 | 167.2 |
| Araldite^{®} DY-E | | 31.8 | 31 8 | 31.8 | 31.8 | 31.8 |
| Amin **1** | | 158.2 | * | - | * | - |
| Amin **2** | | - | 175.3 | - | | - |
| Amin **3** | | - | - | 148.2 | - | - |
| Gaskamine^{®} A-229 | | - | - | - | 102.0 | - |
| Gaskamine^{®} 240 | | - | - | - | - | 103.0 |
| Ancamine^{®} K 54 | | 7.1 | 7.5 | 6.9 | 6.0 | 6.0 |
| Viskosität (10') [Pa·s] | | 0.34 | 0.95 | 0.33 | 0.62 | 0.68 |
| Königshärte [s] | (2d) | 27 | n.m | 10 | n.m. | 127 |
| | (4d) | 108) | n.m. | 27 | 43 | 167 |
| (NK) | (7d) | 146 | 62 | 34 | 76 | 179 |
| | (4w) | 154 | 130 | 36 | 97 | 195 |
| Aspekt (NK) | | schön | schön | schön | schön | schön |
| Königsh. [s] | (7d kalt) | 6 | 4 | 10 | n.m. | 83 |
| | (+2d NK) | 78 | 22 | 27 | 25 | n.b. |
| (8°/80%) | (+7d NK) | 161 | 126 | 52 | 99 | 169 |
| | (+4wNK) | 169 | 150 | 53 | 127 | 174 |
| Aspekt (8°/80%) | | schön | schön | schön | schön | schön |
| Anzahl Markierungen | | 3 | 4 | 4 | 4 | 1 |

**Tabelle 3: Zusammensetzung und Eigenschaften des Beispiels 12 und der Vergleichsbeispiele 11 und 13 bis 16. "I." steht für "leicht"; "schw." steht für "schwach"; "s.schw." steht für "sehr schwach"; "Königsh." steht für "Königshärte"; "Vergl." steht für "Vergleich"**

| **Beispiel** | | **11 (Vergl.)** | **12** | **13 (Vergl.)** | **14 (Vergl.)** | **15 (Vergl.)** | **16 (Vergl.)** |
|---|---|---|---|---|---|---|---|
| Araldite^{®} GY-250 | | 167.2 | 167.2 | 167.2 | 167.2 | 167.2 | 167.2 |
| Araldite^{®} DY-E | | 31.8 | 31.8 | 31.8 | 31.8 | 31.8 | 31.8 |
| EP-Addukt 1 | | 99.3 | - | 74.5 | 74.5 | 74.5 | 74.5 |
| Härter aus Beispiel 1 | | - | 114.1 | - | - | - | - |
| Gaskamine^{®} A-229 | | - | - | 25.5 | - | - | - |
| Gaskamine^{®} 240 | | - | - | - | 25.8 | - | - |
| Amin 2 | | - | - | - | - | 43.8 | - |
| Amin 3 | | - | - | - | - | - | 37.1 |
| Ancamine^{®} K 54 | | 6.0 | 6.3 | 6.0 | 6.0 | 6.4 | 6.4 |
| Viskosität (10') [Pa.s] | | 1.90 | 0.85 | 0.80 | 0.82 | 0.97 | 1.08 |
| Königshärte [s] | (2d) | 181 | 129 | 60 | 80 | 77 | 116 |
| | (4d) | 189 | 171 | 97 | 127 | 123 | 160 |
| (NK) | (7d) | 200 | 182 | 112 | 147 | 148 | 176 |
| | (4w) | 193 | 199 | 130 | 167 | 164 | 193 |
| Aspekt (NK) | | schön | schön | schön | schön | schön | schön |
| Königsh. [s] | (7d kalt) | 101 | 70 | 39 | 52 | 39 | 42 |
| | (+2d NK) | 174 | 119 | 81 | 94 | 90 | 66 |
| (8°/80%) | (+7d NK) | 192 | 160 | 115 | 119 | 147 | 133 |
| | (+4w NK) | 197 | 186 | 125 | 147 | 174 | 186 |
| Aspekt (8°/80%) | | 1. matt | schön | schön | 1. klebrig | schön | schön |
| Anzahl Markierungen | | 4 schw. | 4 s.schw. | 4 schw. | 4 schw. | 4 schw. | 4 s.schw |
| Splitter-Neigung | | mittel | schwach | schwach | mittel | schwach | schwach |

**Tabelle 4: Zusammensetzung und Eigenschaften der Beispielr 18, 20 und 22 und der Vergleichsbeispiele 17, 19 und 21. "weiss" steht für eine weisse Verfärbung der Markierung: "schw," steht für "schwach"; "s.schw." steht für "sehr schwach"; "Königsh." steht für "Königshärte": "Vergl." steht für "Vergleich"**

| **Beispiel** | | **17 (Vergl.)** | **18** | **19 (Vergl.)** | **20** | **21 (Vergl.)** | **22** |
|---|---|---|---|---|---|---|---|
| Araldite^{®} GY-250 | | 167.2 | 167.2 | 167.2 | 167.2 | 167.2 | 167.2 |
| Araldite^{®} DY-E | | 31.8 | 31.8 | 31.8 | 31.8 | 31.8 | 31.8 |
| EP-Addukt **2** (MXDA) | | 106.1 | - | - | - | - | - |
| Härter aus Beispiel **2** | | - | 119.2 | - | - | - | - |
| Polypox^{®} IH 7011 | | - | - | 82.0 | - | - | - |
| Härter aus Beispiel **4** | | - | - | - | 101.1 | - | - |
| Aradur^{®} 3442 | | - | - | - | - | 125.0 | - |
| Härter aus Beispiel **5** | | - | - | - | - | - | 133.4 |
| Ancamine^{®} K 54 | | 6.1 | 6.4 | 5.6 | 6.0 | 6.5 | 6.6 |
| Viskosität (10') [Pa·s] | | 6.96 | 2.39 | 0.93 | 0.66 | 2.82 | 1.03 |
| Königshärte [s] | (2d) | 218 | 195 | 118 | 122 | 52 | 71 |
| | (4d) | 223 | 206 | 108 | 146 | 120 | 112 |
| (NK) | (7d) | 232 | 216 | 150 | 192 | 134 | 132 |
| | (4w) | 231 | 213 | 153 | 211 | 151 | 165 |
| Aspekt (NK) | | schön | schön | schön | schön | schön | schön |
| Königsh. [s] | (7d kalt) | 139 | 98 | 46 | 53 | 70 | 49 |
| | (+2d NK) | 207 | 161 | 89 | 110 | 78 | 64 |
| (8°/80%) | (+7d NK) | 217 | 185 | 141 | 174 | 81 | 123 |
| (+4w | NK) | 216 | 206 | 141 | 191 | 123 | 144 |
| Aspekt (8°/80%) | | matt | matt | schön | schön | schön | schön |
| Anzahl Markierungen | | 4 weiss | 4 schw, | 1 | 1 | 1 schw. | 1 s.schw. |
| Splitter-Neigung | | mittel | schwach | keine | keine | stark | schwach |

## Patentansprüche

1. Härter, geeignet zum Aushärten von Epoxidharzen, umfassend das Amin der Formel (I).

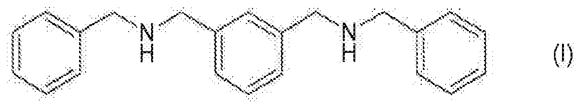

2. Härter gemäss Anspruch 1, **dadurch gekennzeichnet, dass** er weiterhin mindestens ein Polyamin, welches mindestens drei gegenüber Epoxidgruppen reaktive Aminwasserstoffe aufweist, umfasst.

3. Härter gemäss Anspruch 2, **dadurch gekennzeichnet, dass** das Polyamin ausgewählt ist aus der Gruppe bestehend aus 1,3-Pentandiamin, 1,5-Diamino-2-methylpentan, 2-Butyl-2-ethyl-1,5-pentandiamin, 1,6-Hexandiamin, 2,2,4- und 2,4,4-Trimethylhexamethylendiamin, 1,12-Dodecandiamin, 1,3-Diaminocyclohexan, Bis-(4-aminocyclohexyl)methan, Bis-(4-amino-3-methylcyclohexyl)methan, 1-Amino-3-aminomethyl-3,5,5-trimethylcyclohexan, 1,3-Bis-(aminomethyl)cyclohexan, 1,3-Bis-(aminomethyl)-benzol, Bis-hexamethylentriamin, Diethylentriamin, Triethylentetramin, Tetraethylenpentamin, Pentaethylenhexamin und höheren Homologen linearer Polyethylenamine wie Polyethylenpolyamin mit 5 bis 7 Ethylenamin-Einheiten, Dipropylentriamin, N-(2-Aminoethyl)-1,3-propandiamin, N,N'-Bis(3-aminopropyl)ethylendiamin, Polyoxyalkylen-Diaminen und Polyoxyalkylen-Triaminen mit einem Molekulargewicht im Bereich von 200 bis 500 g/mol, Polyamidoaminen, Phenalkaminen, an primären Aminogruppen vollständig oder teilweise alkylierten Verbindungen der genannten Polyamine und Addukten der genannten Polyamine mit Epoxiden und Epoxidharzen.

4. Härter gemäss einem der Ansprüche 2 oder 3, **dadurch gekennzeichnet, dass** das Polyamin mindestens eine sekundäre Aminogruppe aufweist.

5. Härter gemäss einem der Ansprüche 2 bis 4, **dadurch gekennzeichnet, dass** das Verhältnis der Anzahl der gegenüber Epoxidgruppen reaktiven Aminwasserstoffe des Amins der Formel (I) und der Anzahl der gegenüber Epoxidgruppen reaktiven Aminwasserstoffe des Polyamins im Bereich von 0.05 bis 5 liegt.

6. Härter gemäss einem der Ansprüche 2 bis 4, **dadurch gekennzeichnet, dass** das Gewichtsverhältnis des Amins der Formel (I) und des Polyamins im Bereich von 0.2 bis 2 liegt.

7. Härter gemäss einem der Ansprüche 2 bis 6, **dadurch gekennzeichnet, dass** der Gehalt an nicht einbaubaren Verdünnern weniger als 25 Gewichts-% beträgt.

8. Epoxidharz-Zusammensetzung enthaltend
a) mindestens ein Epoxidharz, und
b) mindestens einen Härter gemäss einem der Ansprüche 1 bis 7.

9. Epoxidharz-Zusammensetzung gemäss Anspruch 8, **dadurch gekennzeichnet, dass** das Epoxidharz ein Flüssigharz auf der Basis eines Bisphenols ist.

10. Epoxidharz-Zusammensetzung gemäss einem der Ansprüche 8 oder 9, **dadurch gekennzeichnet, dass** sie weiterhin mindestens einen mindestens eine Epoxidgruppe aufweisenden Reaktivverdünner enthält.

11. Epoxidharz-Zusammensetzung gemäss einem der Ansprüche 8 bis 10, **dadurch gekennzeichnet, dass** sie eine zweikomponentige Zusammensetzung ist, bestehend aus
(i) einer Harz-Komponente enthaltend mindestens ein Epoxidharz und
(ii) einer Härter-Komponente enthaltend den Härter gemäss einem der Ansprüche 1 bis 7.

12. Ausgehärtete Zusammensetzung erhalten aus der Aushärtung einer Zusammensetzung gemäss einem der Ansprüche 8 bis 11.

13. Verwendung einer Epoxidharz-Zusammensetzung gemäss einem der Ansprüche 8 bis 11 als Beschichtung,

14. Artikel erhalten aus der Verwendung gemäss Anspruch 13.
